# EUROPEAN PATENT SPECIFICATION

(11) **EP 3 900 614 B1**
(45) Date of publication and mention of the grant of the patent: **26.04.2023**
(21) Application number: 20214543.9
(22) Date of filing: 16.12.2020
(51) Int. Cl.: A61B 5/00, A61B 5/11

(54) **DEVICE FOR MEASURING KNEE TORQUE IN BEDRIDDEN HUMANS AND METHOD FOR USING THE DEVICE**
VORRICHTUNG ZUR MESSUNG DES KNIEDREHMOMENTS BEI BETTLÄGERIGEN MENSCHEN UND VERFAHREN ZUR VERWENDUNG DER VORRICHTUNG
DISPOSITIF DE MESURE DE COUPLE DE GENOU CHEZ DES PERSONNES ALITÉES ET PROCEDE D'UTILISATION DU DISPOSITIF

(43) Date of publication of application: 27.10.2021
(73) Proprietor: Univerza na Primorskem, Universita del Litorale, 6310 Izola (SI)
(72) Inventor: Sarabon, Nejc, 4000 Kranj (SI)
(74) Representative: Patentni Biro AF d.o.o.

(56) References cited:
- WO-A1-2004/107976
- CN-A- 109 746 940
- US-A1- 2006 224 087
- US-A1- 2008 216 570

## Description

### Field of the invention

The present invention belongs to the field of medical devices, more precisely to the field of devices for measuring muscle function. The object of the invention is a device for measuring knee torque during static contraction in bedridden humans.

### Background of the invention and the technical problem

Musculoskeletal system is an anatomical term that refers to an organ system that is formed by muscles and bones. The musculoskeletal system gives humans the possibility to move through space and perform various physical activities, such as walking and running, and to maintain an upright posture. The ability of the human body to move is largely dependent on the level motor abilities, such as muscle strength, postural balance and flexibility. Muscle strength is defined as the maximum amount of force that a muscle can exert against a resistance. Sufficient level of strength is paramount for the human to be able to perform physical activities. In sport, the strength is developed to the highest possible level to maximize the performance of the athlete. In physical therapy and rehabilitation, the strength is typically aimed to be restored (e.g. after an injury) or improved (e.g. to prevent subsequent injuries). Strength is developed with a training that comprises of repetitions of specific activities, typically involving movements against an external resistance. Strengthening of the muscles is possible with the use of elastic resistance (e.g. elastic bands or tubes), exercise machines, based on pneumatic resistance, or by lifting/moving heavy objects, such as weights.

Direct quantification of muscle strength is impossible in humans. Therefore, the procedures for assessing individual's strength instead measure the torque that the muscles generate around a given joint. For example, the extension torque generated in the knee joint is accepted as a measure of the strength of the muscles that generate forces that are translated to extension torque. Joint torque is typically measured with devices called dynamometers, which are comprised of one or more torque or forces sensors and a mechanical structure that enable appropriate positioning of the person. The dynamometers may enable assessing of static strength (i.e. produced torque while the body is not moving) or dynamic strength (i.e. produced torque while the body is moving). Most often, scientists and clinicians are interested in maximal voluntary torque, which is defined as the torque that is produced by the subject with his/her maximal effort. In some cases, another measure of interest includes the involuntary torque (e.g., evoked by electrical stimulation).

The knee joint is one of the most commonly assessed joints in terms of strength in sport sciences and medicine, rehabilitation, gerontology and other human health sciences. This is attributable to the convenience of the knee strength measurements, as well as the good validity and reliability. Various devices have been developed to enable knee torque measurements. Most devices are constructed in a way to allow the subject to be seated, such as devices disclosed in documents US4702108A, US4727860A and CA2883174A1. Moreover, some of the devices allow for the measurements in lying (prone or supine) positions. One of the main limitations of the state-of-the-art dynamometers their weight and size, which makes their transport very limited. On the other hand, transportable devices, such as hand-held dynamometers, are not highly reliable. Therefore, valid and reliable strength measurements are not accessible to some populations, notably for the patients who are hospitalized and are unable to get of their beds.

The aim of the present invention is to provide a transportable dynamometer device that enables the measurement of knee joint torque in a lying (supine) position. The design of the dynamometer should allow easy set-up of the patients without them leaving the bed.

### State of the art

Several devices that allow knee torque measurements in the seated position have been developed and disclosed. The device disclosed in US4702108A is characterized by complex mechanical frame, which enables knee torque measurements in a seated position whilst the subject is fixated. The position of the leg within the device can be configured, which enables that the knee torque is assessed at different positions (i.e. different angles) of the knee. Another similar device has been disclosed in US4727860A. This device combines features that enable both exercise and testing, including the assessment of the knee torque, but not in the lying position. Similarly, the device disclosed in CA2883174A1 enables knee torque measurements and is relatively low-cost and easy-to-transport, however, the measurements cannot be performed in a lying position.

Several dynamometers are designed in a way to enable measurements of the strength of more than one joint, such as the ones disclosed in documents US2590055A, US4727860A, US5722937A, US2006224087, however, none of these enable measurements to be done in a lying position, let alone, none of these devices can be used with or integrated within a hospital bed. Patent application US2006224087 discloses a complicated construction of a device for measuring knee-torque in a sitting position. Among other components necessary for the stability of the device, the latter has a base plate on which a tested person sits and a dynamometer framework comprising two elements with an intersection at the knee joint. These two elements are pivotably connected and one is arranged to receive and fix the shin of the tested person, while the other element is arranged to be located at the side of the thigh of the tested person. The position of the user is fixed with a belt, however, this is not efficient in preventing movement of the hips, which is crucial for ensuring reliable measurement.

The present invention is functionally similar to the device disclosed in patent US2590055A. This device enables torque measurements for different joints, and enables the measurements to be performed in different positions, including lying. However, the said device is complex, heavy and non-transportable.

### Description of the solution of the technical problem

The present invention is based on a large body of scientific works that demonstrate the utility, validity and reliability of devices for measurement of knee joint torque. The main characteristic of the invention is that its construction is arranged for measurements in lying position, which is particularly useful for patients not able to be seated or put in any other position. Reliable measurements of knee strength in bedridden patients are important to evaluate their status or rehabilitation progress, which enables hospital and/or nursing home staff to decide on further treatments and/or rehabilitation.

The invention is best summarized by the device of claim 1 and the method of claim 13. Advantageous variations are according to dependent claims 2-12, 14, 15.

The device for measuring knee torque during static contraction in bedridden humans will be described in further detail based on exemplary embodiments and figures, which show:
- Figure 1: The device shown as folded (a) and unfolded (b)
- Figure 2: The device shown as to be used for the measurement of the right (a and the left (b) leg
- Figure 3: Close up of the shorter elements, showing how the leg is secured to the device (a), with the elements also depicted without the leg (b)
- Figure 4: Close up of the electronic unit
- Figure 5: A representative image of the suggested position for measurements

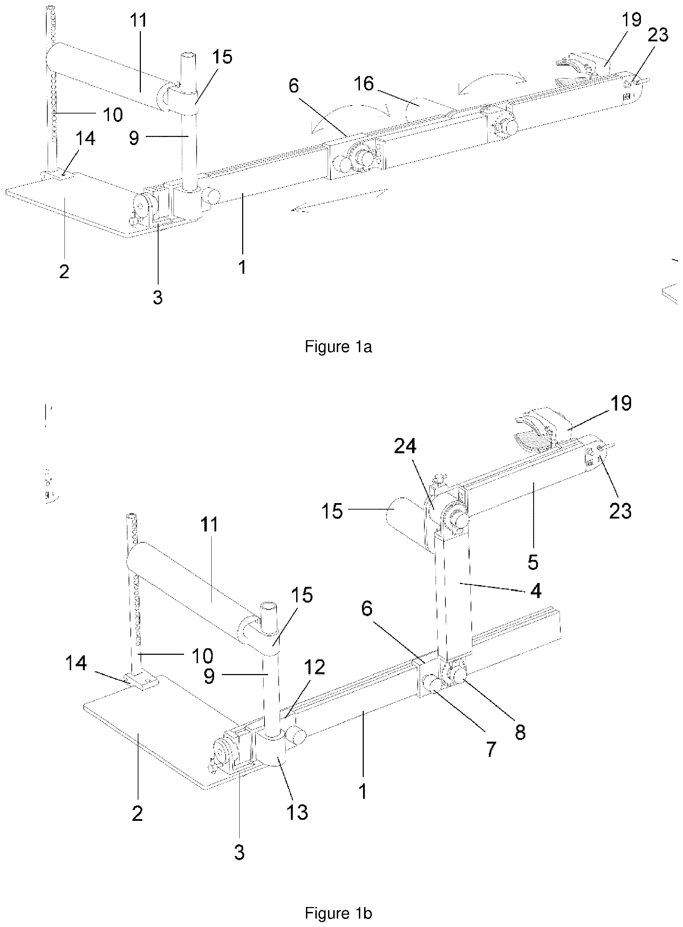

The device for measuring knee torque during static contraction in bedridden patients according to a possible embodiment as shown in figure 1 comprises the following components:
- a base plate 2, which is arranged to be positioned under the patient's hips,
- a fixation frame, intended for fixating the device to the patient, comprising:
   - two vertical poles 9,10, one on both sides of the base plate 2;
   - a horizontal height-adjustable pole 11 for forming a rectangle together with the base plate 2 and the vertical poles 9,10, which is arranged to safely stabilize the patient's hips;
- a mechanical dynamometer framework, comprising:
   - a long horizontal element attached to any side on one end of the base plate,
   - two shorter elements connected in series next to the longer horizontal element opposite from the base plate,
- a torque sensor 24 installed at the junction of the two shorter elements;
- a brace for fixation of the shin, located at the distal part of the second shorter element;
- a fixation belt 18 for the knee, located at the junction of the short elements 4,5;
- a cylindrical element 16 for knee support, located at the junction of the short elements 4,5;
- main electronic unit 23 for controlling measurements and the torque sensor 24 readings.

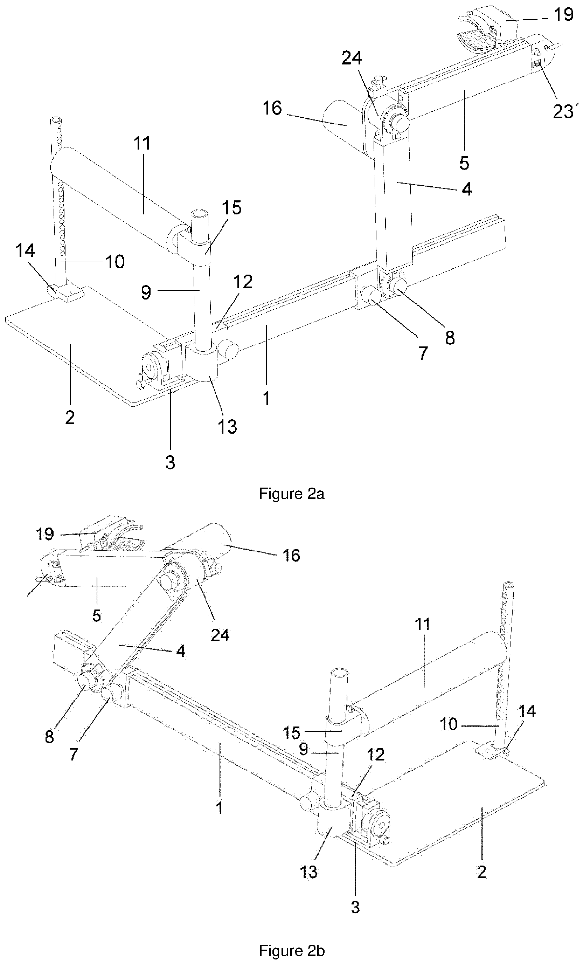

The angles between the long 1 and shorter elements 4, 5 of the dynamometer framework may be adjusted according to patient's body measurements. As the longer horizontal element 1 may be rotated with respect to the base plate 2, measurements on both legs are possible as shown in Figure 2.

The length and width of the base plate 2 are 60 and 30 cm, respectively, with small deviations allowed (< 5 cm). The long horizontal element 1 is approximately 1 m long, while the shorter elements (4, 5) of the dynamometer framework are approximately 0.5 m in length. The long horizontal element 1 is attached to the base plate 2 with a locking unit, which includes a rotatable mechanism 3 that can be unlocked with a screw to allow rotation of the long horizontal element 1 around the long axis of the base plate 2 to achieve the two opposite configurations shown in Figure 2. The proximal short element 4 attaches to the longer horizontal element, and the distal short element 5 attaches to the proximal short element. The proximal attachment block 6 between the horizontal long element 1 and the proximal short element 4 is movable across the length of the horizontal long element 1. Specifically, the proximal attachment block 6 is inserted on the horizontal longer element 1 in the way that it can slide along it as on the rail. The proximal attachment block is unlocked by releasing the screw 7. The proximal shorter element 4 is attached to the proximal attachment block 6. This junction involves one of the screws and allows the rotation of the proximal shorter element. When the desired position is reached, the screw 8 is tightened to secure the short element in place. The proximal 4 and distal 5 short elements are attached together in the same way, enabling rotation and fixation. The said joints between the elements enable to change the configuration of the device between folded (Figure 1b) and unfolded state (Figure 1a).

The fixation framework is attached to the base plate and consists of two vertical poles, one 9 on the side of the main dynamometer frame and one on the opposite side 10. The vertical poles are connected with the horizontal pole 11. The first vertical pole 9 is attached to the long horizontal element 1 in the same manner as the proximal short element 4. The attachment block for the pole 12 is the same as the proximal attachment block 6. On the attachment block for the pole 12, there is an additional supporting element 13, into which the fist vertical pole 9 is inserted. This enables the pole to be rotated in the transversal plane. This is necessary, similar to the rotation of the long horizontal element, to accommodate the configuration of the device for the measurement of the opposite leg. The second vertical pole 10 is not rotatable, but it can be removed from the base plate 2 when needed. The position of the second vertical pole may be fixed with a locking pin or a suitably shaped locking mechanism. The horizontal pole 11 is positioned perpendicularly to the vertical poles 9,10. The horizontal pole 11 must be padded to prevent discomfort of the patients. Namely, the horizontal pole 11 is pressed against the users hips to ensure the stability of the hip/pelvic area during the measurements. On the side of the first vertical pole 9, the horizontal pole 11 is attached via a connecting holding element 15. The said connecting element 15 is directly attached to the horizontal pole 11. The vertical pole 9 passes through the opening that is cut into the holding element 15 with a locking pin. The second vertical pole 10 has several intends, intended for securing of the horizontal element 11.

At the junction of the two short elements 4,5 of the main dynamometer framework, an additional cylindrical element 16 is provided that ensures the stability of the knee joint during the testing. An additional aluminium plate 17 is attached at the inner side of the proximal short element 4. The aluminium plate 17 covers the proximal short element 4 in its distal part, starting the junction of the short elements and covering additional 10 cm of the proximal short element 4. The cylindrical element 16 is attached to the aluminium plate 17. The plate is removable and may be mounted also on the opposite side of the junction, which needs to be done when the testing leg is changed. The knee belt 18 is provided to secure the leg against the cylindrical element 16.

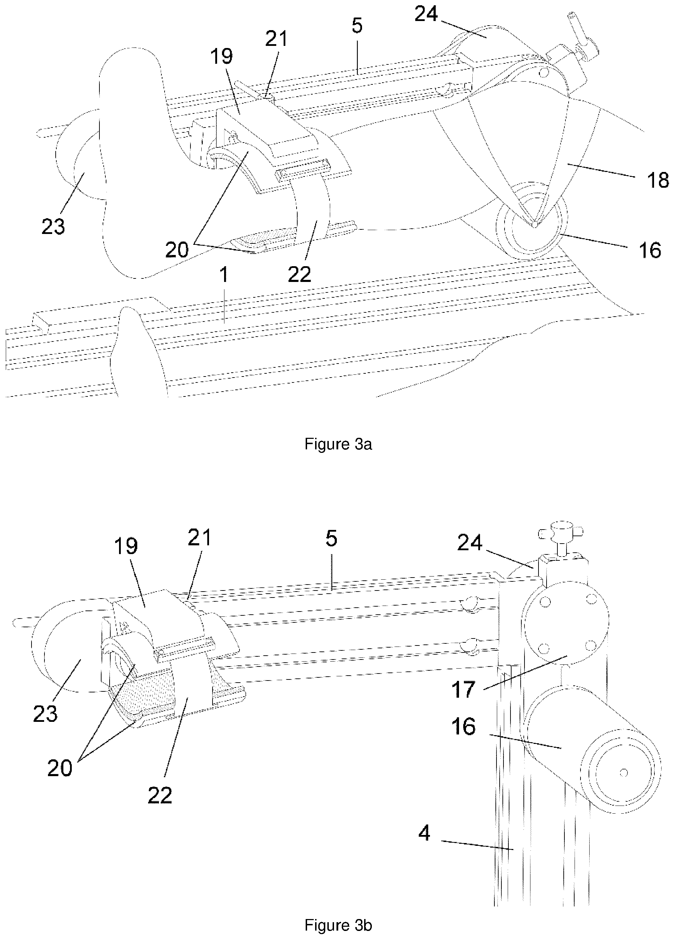

The shin brace is attached to the distal short element 5. The attachment point of the shin brace is movable along the distal short element 5. The shin brace consists of a basic frame 19 and two fixation pads 20. The basic frame 19 is in contact with the distal short element 5. The point attachment between the two structures may be changed, by releasing the screwing mechanism 21. Within the basic frame 19, two pads 20 are positioned. The said pads 20 are facing each other with their concave sides and are intended to grasp the subject's shin as shown in figure Figure 3a. Additional belt 22 is provided, which makes possible to tightly fixate the pads 20 against the subject's shin.

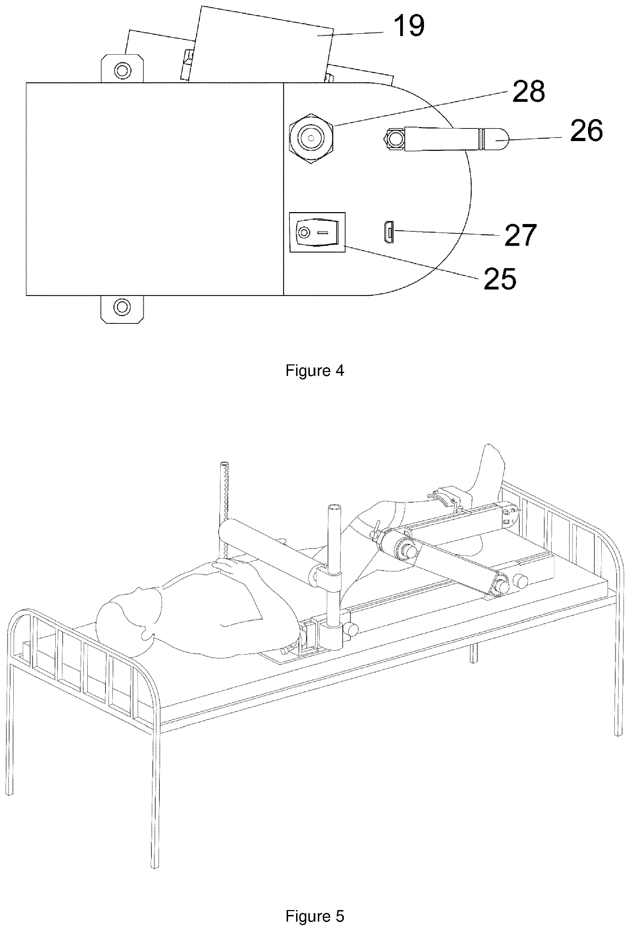

As shown in Figure 4 the main electronic unit 23 is located at the end of the distal short element. It receives signal input from the torque sensor 24, which is located in the junction between the two short elements 4,5. The main electronic unit comprises:
- an on/off switch 25,
- input/output ports 27 such as a USB port,
- a wireless antenna 26, which enables to transmit the data wirelessly, and
- a raw signal out connector (28).

The device as described above ensures high accuracy measurements of the joint torque, especially in patients constrained to their beds at hospitals or nursing homes, but also elsewhere. Figure 5 depicts the typical position of the patient when prepared for performing measurements, wherein the horizontal pole 11 of the fixation frame is placed on the patient's hips, patient's knee and shin are secured with the knee belt and the shin brace and the distal short element 5 of the dynamometer framework is parallel to the long element 1 of the dynamometer framework.

## Claims

1. A device for measuring knee torque during static voluntary or stimulated contraction in bedridden humans, for performing measurements on a knee of a leg of a human placed in supine position, wherein the device comprises:
- a base plate (2), which is arranged to be positioned under the patient's hips in the supine position, wherein further elements and their orientation are defined with respect to an orientation of the device in which the base plate is horizontally oriented;
- fixation means connected to the base plate (2) for fixation of the device to the patient hips for ensuring reliable measurements;
- a mechanical dynamometer framework, comprising:
∘ a long element (1) attached at a first end to one side of the base plate (2), such that the long element extends away from the base plate,
∘ two shorter elements (4,5) connected to the longer element (1) at the second end of the longer element, , wherein the first shorter element (4) is on one end slidably and rotatably mounted on the longer horizontal element (1) and the second shorter element (5) is rotatably mounted the other end of the first shorter element (4),
wherein the long element (1) is arranged to be placed on the outer side of the tested leg;
- a torque sensor installed at the junction of the two shorter elements (4,5);
- a brace for fixation of the shin, located at the free end of the second shorter element (5);
- a fixation belt (18) for the knee, located at the junction of the short elements (4,5);
- a cylindrical element (16) for knee support, located at the junction of the short elements (4,5);
- a main electronic unit (23) for processing data sent from the torque sensor.

2. The device for measuring knee torque during static contraction in bedridden humans according to claim 1, wherein fixation means may be an adjustable belt or preferably a fixation frame, which comprises:
- two vertical poles (9,10), one on both sides of the base plate (2);
- a horizontal height-adjustable pole (11) for forming a rectangle together with the base plate (2) and the vertical poles (9,10), which is arranged to safely stabilize the patient's hips, wherein one of the vertical poles (9,10) can be reversibly removed and installed for easier positioning of the patient on the device,
so that the three poles (9,10,11) form a rectangle parallel to transversal cross-section of the patient's body.

3. The device for measuring knee torque during static contraction in bedridden humans according to claim 2, wherein the horizontal pole (11) is preferably equipped with two shorter vertical elements at the sides, said vertical elements being perpendicular to the horizontal pole and parallel to the vertical poles; the shorter vertical elements are slidably mounted in the horizontal pole (11), so that the distance between them can be adjusted; and wherein these shorter vertical elements are provided to fix the patient's hips but at the same time allow needed space in case flexion and extension of the knee have to be stimulated.

4. The device for measuring knee torque during static contraction in bedridden humans according to any of the preceding claims, **characterized in that** angles between the long horizontal element (1) and the proximal shorter element (4) as well as between the two shorter elements (4,5) can be adjusted with suitable mountings.

5. The device for measuring knee torque during static contraction in bedridden humans according to any of the preceding claims, **characterized in that**:
- a first mounting is between the long horizontal element (1) and the proximal shorter element (4) is designed as a proximal attachment block (6), which can slide along at least one groove or rail provided in the long horizontal element (1), wherein movement of the said block (6) is controlled with a safety pin or screw (7) arranged to fall into a suitable hole in the long element (1), so that sliding motion of the proximal shorter element (4) is disabled when the pin (7) sits in the said hole; and **in that**
- the proximal attachment block (6) also ensures rotation of the proximal short element (4) from the horizontal position into the vertical position needed for measurements, wherein the angle between the horizontal element (1) and the proximal shorter element (4) may be adjusted by rotating the shorter element (4) and locking its position with a second pin (8) corresponding to a second hole.

6. The device for measuring knee torque during static contraction in bedridden humans according to any of the preceding claims, **characterized in that** the first and second short elements (4,5) are attached in a second mounting, enabling rotation and fixation with pins or screws.

7. The device for measuring knee torque during static contraction in bedridden humans according to any of the preceding claims, **characterized in that** the longer horizontal element (1) is attached to the base plate (2) with a locking unit, which includes a rotatable mechanism that can be unlocked to allow rotation of the long horizontal element (1) around the long axis of the base plate (2).

8. The device for measuring knee torque during static contraction in bedridden humans according to any of the preceding claims, **characterized in that**
- the base plate (2) is made of hard, non-compliant material, such as aluminium or other metal materials, wherein the preferred length and width of the base plate (2) are between 50 and 70 cm, optimally 60 cm, and between 25 and 35 cm, optimally 30 cm, respectively; and **in that**
- the long horizontal element (1) is approximately 1 m long and is made of a single aluminium profile, while the shorter elements (4,5) are aluminium profiles measuring 0.5 m in length.

9. The device for measuring knee torque during static contraction in bedridden humans according to any of the preceding claims, **characterized in that** the knee support (16) may be fixed or may be preferably movable using known rotatable mountings so that a suitable knee angle can be set; and **in that** the knee support (16) is provided with the belt (18) attached near the junction of the two shorter elements (4,5) for securing the knee tightly against the cylindrical support element (16).

10. The device for measuring knee torque during static contraction in bedridden humans according to any of the preceding claims, **characterized in that** the shin brace has two slightly curved surfaces, which are facing each other with their concave sides, wherein the distance between the surfaces is adjustable, which allows for the shin to be grasped at the distal part just above the ankle.

11. The device for measuring knee torque during static contraction in bedridden humans according to any of the preceding claims, **characterized in that** the torque sensor can be any suitable to measure torque, which is arranged to send data to the main electronic unit during measurement, wherein the said main electronic (23) unit comprises:
- an on/off switch (25),
- input/output ports (27) such as a USB port,
- a wireless antenna (26), which enables to transmit the data wirelessly, and
- a raw signal out connector (28),
and is arranged to amplify signals from the torque sensor, performing analogue-to-digital conversion, acquiring the data at 1000 Hz and transmitting the signals via a Bluetooth module to a computer.

12. The device for measuring knee torque during static contraction in bedridden humans according to any of the preceding claims, **characterized in that** the device may further comprise additional plates connected to the long horizontal element of the dynamometer framework.

13. A method for using the device according to any of the preceding claims, **characterized in that** a patient is positioned and fixated as follows:
- the patient's hip/pelvic area is resting on the base plate (2), and the patient is lying supine,
- the fixation frame (9,10,11) is tightly securing the patients hips against the base plate,
- the longer horizontal element (1) continues horizontally from the end of the base plate (2) on the side of the tested leg,
- the two shorter elements (4,5) are arranged in a triangle shape, wherein the first shorter element (4) starts at the distal part of the longer frame element, and points towards the patient's upper body and the junction of the two shorter elements is at the knee joint, while the second shorter element (5) is pointing away from the subject,
- the distal part of the shin is secured within the fixation brace,
- the knee is secured against the cylindrical element (16) with fixation belt (18),
- patient's hip and knee joints are in a flexed position (around 45° each, with possible adjustments).

14. A method using the device according to the preceding claim, **characterized in that** measurements are performed in the following way:
- the patient flexes and extends the knee or muscle activity is triggered externally using known methods for stimulation of groin nerve or thigh muscle, wherein the sensor measures the created torque,
- flexion and extension may be repeated several times in order to ensure a more reliable measurement,
- the data from the sensor are sent to the main electronic unit and optionally a computer for processing and analysis.

15. The device according to any of claims 1-12, **characterized in that** it is embedded into a frame of a bed.

## Patentansprüche

1. Vorrichtung zur Messung des Kniedrehmoments bei bettlägerigen Menschen während statischer freiwilliger oder stimulierter Kontraktion zum Durchführen von Messungen an einem Knie eines Beins eines Menschen, der sich in Rückenlage befindet, wobei die Vorrichtung Folgendes umfasst:
- eine Bodenplatte (2), die angeordnet ist, um in Rückenlage unter den Hüften des Patienten positioniert zu werden, wobei weitere Elemente und ihre Ausrichtung in Bezug auf eine Ausrichtung der Vorrichtung definiert sind, bei der die Bodenplatte horizontal ausgerichtet ist;
- mit der Bodenplatte (2) verbundene Befestigungsmittel zur Befestigung der Vorrichtung an den Hüften des Patienten, um zuverlässige Messungen sicherzustellen;
- einen mechanischen Dynamometerrahmen, umfassend:
∘ ein langes Element (1), das an einem ersten Ende an einer Seite der Bodenplatte (2) befestigt ist, sodass sich das lange Element von der Bodenplatte weg erstreckt,
∘ zwei kürzere Elemente (4, 5), die mit dem längeren Element (1) am zweiten Ende des längeren Elements verbunden sind, wobei das erste kürzere Element (4) an einem Ende verschiebbar und drehbar an dem längeren horizontalen Element (1) angebracht ist, und das zweite kürzere Element (5) am anderen Ende des ersten kürzeren Elements (4) drehbar montiert ist,
wobei das lange I-Element (1) angeordnet ist, um auf der Außenseite des getesteten Beins platziert zu werden;
- einen Drehmomentsensor, der an der Verbindungsstelle der beiden kürzeren Elemente (4, 5) installiert ist;
- eine am freien Ende des zweiten kürzeren Elements (5) angeordnete Schiene zur Fixierung des Schienbeins;
- einen Befestigungsgurt (18) für das Knie, der sich an der Verbindungsstelle der kurzen Elemente (4, 5) befindet;
- ein zylindrisches Element (16) zur Kniestütze, das sich an der Verbindungsstelle der kurzen Elemente (4, 5) befindet;
- eine elektronische Haupteinheit (23) zum Verarbeiten von Daten, die von dem Drehmomentsensor gesendet werden.

2. Vorrichtung zum Messen des Kniedrehmoments bei bettlägerigen Menschen während statischer Kontraktion nach Anspruch 1, wobei das Befestigungsmittel ein einstellbarer Gurt oder vorzugsweise ein Befestigungsrahmen sein kann, der Folgendes umfasst:
- zwei vertikale Stangen (9,10), eine auf beiden Seiten der Grundplatte (2);
- eine horizontal höhenverstellbare Stange (11) zum Bilden eines Rechtecks zusammen mit der Grundplatte (2) und den vertikalen Stangen (9, 10), die angeordnet ist, um die Hüften des Patienten sicher zu stabilisieren, wobei eine der vertikalen Stangen (9, 10) zur einfacheren Positionierung des Patienten auf der Vorrichtung reversibel entfernt und installiert werden kann,
sodass die drei Stangen (9, 10, 11) ein Rechteck parallel zum transversalen Querschnitt des Körpers des Patienten bilden.

3. Vorrichtung zum Messen des Kniedrehmoments bei bettlägerigen Menschen während statischer Kontraktion nach Anspruch 2, wobei die horizontale Stange (11) vorzugsweise mit zwei kürzeren vertikalen Elementen an den Seiten ausgestattet ist, wobei die vertikalen Elemente senkrecht zur horizontalen Stange und parallel zu den vertikalen Stangen sind; die kürzeren vertikalen Elemente verschiebbar in der horizontalen Stange (11) montiert sind, sodass der Abstand zwischen ihnen eingestellt werden kann; und wobei diese kürzeren vertikalen Elemente vorgesehen sind, um die Hüften des Patienten zu fixieren, aber gleichzeitig den erforderlichen Platz bieten, falls Beugung und Streckung des Knies stimuliert werden müssen.

4. Vorrichtung zum Messen des Kniedrehmoments bei bettlägerigen Menschen während statischer Kontraktion nach einem der vorstehenden Ansprüche, **dadurch gekennzeichnet, dass** Winkel zwischen dem langen horizontalen Element (1) und dem proximalen kürzeren Element (4) sowie zwischen den beiden kürzeren Elementen (4,5) mit geeigneten Halterungen angepasst werden können.

5. Vorrichtung zum Messen des Kniedrehmoments bei bettlägerigen Menschen während statischer Kontraktion nach einem der vorstehenden Ansprüche, **dadurch gekennzeichnet, dass**:
- eine erste Halterung zwischen dem langen horizontalen Element (1) und dem proximalen kürzeren Element (4) als proximaler Befestigungsblock (6) ausgebildet ist, der entlang mindestens einer in dem langen horizontalen Element (1) vorgesehenen Nut oder Schiene gleiten kann, wobei die Bewegung des Blocks (6) mit einem Sicherheitsstift oder einer Sicherheitsschraube (7) gesteuert wird, der/die angeordnet ist, um in ein geeignetes Loch in dem langen Element (1) zu fallen, sodass eine Gleitbewegung des proximalen kürzeren Elements (4) verhindert wird, wenn der Stift (7) in dem Loch sitzt; und dadurch, dass
- der proximale Befestigungsblock (6) auch eine Drehung des proximalen kurzen Elements (4) von der horizontalen Position in die für Messungen benötigte vertikale Position gewährleistet, wobei der Winkel zwischen dem horizontalen Element (1) und dem proximalen kürzeren Element (4) durch Drehen des kürzeren Elements (4) und Verriegeln seiner Position mit einem zweiten Stift (8), der einem zweiten Loch entspricht, eingestellt werden kann.

6. Vorrichtung zum Messen des Kniedrehmoments bei bettlägerigen Menschen während statischer Kontraktion nach einem der vorstehenden Ansprüche, **dadurch gekennzeichnet, dass** das erste und das zweite kurze Element (4, 5) in einer zweiten Halterung befestigt sind, die eine Drehung und Fixierung mit Stiften oder Schrauben ermöglicht.

7. Vorrichtung zum Messen des Kniedrehmoments bei bettlägerigen Menschen während statischer Kontraktion nach einem der vorstehenden Ansprüche, **dadurch gekennzeichnet, dass** das längere horizontale Element (1) an der Bodenplatte (2) mit einer Verriegelungseinheit befestigt ist, die einen drehbaren Mechanismus einschließt, der entriegelt werden kann, um eine Drehung des langen horizontalen Elements (1) um die lange Achse der Bodenplatte (2) zu ermöglichen.

8. Vorrichtung zum Messen des Kniedrehmoments bei bettlägerigen Menschen während statischer Kontraktion nach einem der vorstehenden Ansprüche, **dadurch gekennzeichnet, dass**
- die Bodenplatte (2) aus hartem, nicht nachgiebigem Material, wie Aluminium oder anderen Metallmaterialien, hergestellt ist, wobei die bevorzugte Länge und Breite der Bodenplatte (2) zwischen 50 und 70 cm, optimalerweise 60 cm, und zwischen 25 und 35 cm, optimalerweise 30 cm, beträgt; und dass
- das lange horizontale Element (1) ungefähr 1 m lang ist und aus einem einzigen Aluminiumprofil hergestellt ist, während die kürzeren Elemente (4, 5) Aluminiumprofile mit einer Länge von 0,5 m sind.

9. Vorrichtung zum Messen des Kniedrehmoments bei bettlägerigen Menschen während statischer Kontraktion nach einem der vorstehenden Ansprüche, **dadurch gekennzeichnet, dass** die Kniestütze (16) fest oder vorzugsweise unter Verwendung bekannter drehbarer Halterungen beweglich sein kann, sodass ein geeigneter Kniewinkel eingestellt werden kann; und dadurch, dass die Kniestütze (16) mit dem Gurt (18) versehen ist, der nahe der Verbindungsstelle der zwei kürzeren Elemente (4, 5) befestigt ist, um das Knie eng an dem zylindrischen Stützelement (16) zu befestigen.

10. Vorrichtung zum Messen des Kniedrehmoments bei bettlägerigen Menschen während statischer Kontraktion nach einem der vorstehenden Ansprüche, **dadurch gekennzeichnet, dass** die Schienbeinstütze zwei leicht gekrümmte Oberflächen aufweist, die mit ihren konkaven Seiten einander zugewandt sind, wobei der Abstand zwischen den Oberflächen einstellbar ist, wodurch das Schienbein am distalen Teil direkt über dem Knöchel erfasst werden kann.

11. Vorrichtung zum Messen des Kniedrehmoments bei bettlägerigen Menschen während statischer Kontraktion nach einem der vorstehenden Ansprüche, **dadurch gekennzeichnet, dass** der Drehmomentsensor jeder beliebige geeignete Drehmomentsensor sein kann, der angeordnet ist, um während der Messung Daten an die Hauptelektronikeinheit zu senden, wobei die elektronische Haupteinheit (23) Folgendes umfasst:
- einen Ein-/Aus-Schalter (25)
- Eingangs-/Ausgangsanschlüsse (27), wie einen USB-Anschluss,
- eine drahtlose Antenne (26), die es ermöglicht, die Daten drahtlos zu übertragen, und
- einen Rohsignal-Ausgangsanschluss (28),
und angeordnet ist, um Signale von dem Drehmomentsensor zu verstärken, eine Analog-Digital-Wandlung durchzuführen, die Daten bei 1000 Hz zu erfassen und die Signale über ein Bluetooth-Modul an einen Computer zu übertragen.

12. Vorrichtung zum Messen des Kniedrehmoments bei bettlägerigen Menschen während statischer Kontraktion nach einem der vorstehenden Ansprüche, **dadurch gekennzeichnet, dass** die Vorrichtung ferner zusätzliche Platten umfassen kann, die mit dem langen horizontalen Element des Dynamometerrahmens verbunden sind.

13. Verfahren zur Verwendung der Vorrichtung nach einem der vorstehenden Ansprüche, **dadurch gekennzeichnet, dass** ein Patient wie folgt positioniert und fixiert werden kann:
- der Hüft-/Beckenbereich des Patienten ruht auf der Bodenplatte (2) und der Patient liegt in Rückenlage,
- der Fixierungsrahmen (9, 10, 11) sichert die Hüfte des Patienten fest an der Grundplatte,
- das längere horizontale Element (1) setzt sich horizontal vom Ende der Bodenplatte (2) auf der Seite des getesteten Beins fort,
- die beiden kürzeren Elemente (4, 5) sind in einer Dreiecksform angeordnet, wobei das erste kürzere Element (4) am distalen Teil des längeren Rahmenelements beginnt und zum Oberkörper des Patienten gerichtet ist und die Verbindungsstelle der beiden kürzeren Elemente am Kniegelenk ist, während das zweite kürzere Element (5) vom Subjekt weg zeigt,
- der distale Teil des Schienbeins wird in der Fixationsschiene gesichert,
- das Knie wird mit Fixiergurt (18) am zylindrischen Element (16) gesichert,
- die Hüft- und Kniegelenke des Patienten sind in einer gebeugten Position (jeweils etwa 45°, mit möglichen Anpassungen).

14. Verfahren unter Verwendung der Vorrichtung nach dem vorstehenden Anspruch, **dadurch gekennzeichnet, dass** Messungen auf folgende Weise durchgeführt werden:
- der Patient beugt und streckt das Knie oder die Muskelaktivität wird extern unter Verwendung bekannter Verfahren zur Stimulation des Leistennervs oder des Oberschenkelmuskels ausgelöst, wobei der Sensor das erzeugte Drehmoment misst,
- Beugung und Streckung können mehrmals wiederholt werden, um eine zuverlässigere Messung zu gewährleisten,
- die Daten vom Sensor werden zur Verarbeitung und Analyse an die Hauptelektronikeinheit und optional an einen Computer gesendet.

15. Vorrichtung nach einem der Ansprüche 1 bis 12, **dadurch gekennzeichnet, dass** sie in einen Rahmen eines Bettes eingebettet ist.

## Revendications

1. Un appareil de mesure de couple du genou pendant les contractions statiques volontaires ou stimulées chez des êtres humains alités pour la prise de mesures sur le genou d'une jambe d'une personne allongée sur le dos, où l'appareil comprend :
- une plaque d'embase (2), qui est disposée de façon à être placée sous les hanches du patient couché sur le dos, où des éléments supplémentaires et leur orientation sont définis en relation avec l'orientation de l'appareil dans laquelle la plaque d'embase est orientée horizontalement ;
- un moyen de fixation raccordé à la plaque d'embase (2) pour la fixation de l'appareil aux hanches du patient, pour garantir des mesures fiables ;
- un cadre à dynamomètre mécanique, comprenant :
∘ un élément long (1) fixé à une première extrémité sur un côté de la plaque d'embase (2), de façon à ce que l'élément long se déploie en s'éloignant de la plaque d'embase,
∘ deux éléments plus courts (4, 5) raccordés à l'élément plus long (1) au niveau de la seconde extrémité de l'élément long, où le premier élément plus court (4) est monté sur une extrémité de façon à pouvoir glisser et pivoter sur l'élément horizontal plus long (1) et le second élément plus court (5) est monté de façon à pouvoir pivoter sur l'autre extrémité du premier élément plus court (4),
où l'élément long (1) est disposé de façon à être placé sur le côté externe de la jambe testée ;
- un capteur de couple est installé à la jonction des deux éléments plus courts (4, 5) ;
- une attelle pour la fixation du tibia, située à l'extrémité libre du second élément plus court (5) ;
- une sangle de fixation (18) pour le genou, située à la jonction des éléments courts (4, 5) ;
- un élément cylindrique (16) pour soutenir le genou, situé à la jonction des éléments courts (4, 5) ;
- un boîtier électronique principal (23) pour le traitement des données envoyées par le capteur de couple.

2. L'appareil de mesure de couple du genou pendant les contractions statiques chez les êtres humains alités selon la revendication 1, où le moyen de fixation peut être une sangle ajustable ou de préférence un cadre de fixation, qui comprend :
- deux tiges verticales (9, 10), une de chaque côté de la plaque d'embase (2) ;
- une tige horizontale ajustable en hauteur (11) pour former un rectangle avec la plaque d'embase (2) et les tiges verticales (9, 10), qui est disposée de façon à stabiliser de façon sûre les hanches du patient, où une des tiges verticales (9, 10) peut être retirée et installée de manière réversible pour un positionnement plus facile du patient sur l'appareil,
de façon à ce que les trois tiges (9, 10, 11) forment un rectangle parallèle à la coupe transversale du corps du patient.

3. L'appareil de mesure de couple du genou pendant les contractions statiques chez les êtes humains alités selon la revendication 2, où la tige horizontale (11) est de préférence équipée de deux éléments verticaux plus courts sur les côtés, lesdits éléments verticaux étant perpendiculaires à la tige horizontale et parallèles aux tiges verticales ; les éléments verticaux plus courts sont montés sur la tige horizontale (11) de façon à pouvoir glisser, de sorte que la distance entre eux puisse être ajustée ; et où ces éléments verticaux plus courts sont présents pour fixer les hanches du patient mais en même temps permettent l'espace nécessaire au cas où la flexion et l'extension du genou doivent être stimulées.

4. L'appareil de mesure de couple du genou pendant les contractions statiques chez les êtres humains alités selon l'une quelconque des revendications précédentes, **caractérisé par le fait que** les angles entre l'élément horizontal long (1) et l'élément plus court proximal (4) ainsi qu'entre les deux éléments plus courts (4, 5) peuvent être ajustés à l'aide de supports de fixation appropriés.

5. L'appareil de mesure de couple du genou pendant les contractions statiques chez les êtres humains alités selon l'une quelconque des revendications qui précèdent, **caractérisé par le fait que** :
- un premier support de fixation situé entre l'élément horizontal long (1) et l'élément plus court proximal (4) est conçu comme un bloc de fixation proximal (6), qui peut glisser le long d'au moins une rainure ou d'un rail présent sur l'élément horizontal long (1), où le mouvement dudit bloc (6) est contrôlé par une goupille ou une vis de sécurité (7) disposée pour s'enfoncer dans un trou approprié sur l'élément long (1), de sorte que le mouvement de glissement de l'élément plus court proximal (4) est désactivé quand la goupille (7) repose dans ledit trou ; et en ce que
- le bloc de fixation proximal (6) permet aussi la rotation de l'élément court proximal (4) depuis la position horizontale vers la position verticale nécessaire pour les prises de mesure, où l'angle entre l'élément horizontal (1) et l'élément plus court proximal (4) peut être ajusté en faisant pivoter l'élément plus court (4) et en verrouillant sa position avec une seconde goupille (8) correspondant à un second trou.

6. L'appareil de mesure de couple du genou pendant les contractions statiques chez les êtres humains alités selon l'une quelconque des revendications qui précèdent, **caractérisé par le fait que** les premier et second éléments courts (4, 5) sont fixés sur un second support, permettant la rotation et la fixation avec des goupilles ou vis.

7. L'appareil de mesure de couple du genou pendant les contractions statiques chez les êtres humains alités selon l'une quelconque des revendications qui précèdent, **caractérisé par le fait que** l'élément horizontal plus long (1) est fixé à la plaque d'embase (2) par un dispositif de verrouillage, qui comprend un mécanisme rotatif qui peut être déverrouillé pour permettre la rotation de l'élément long horizontal (1) autour de l'axe long de la plaque d'embase (2).

8. L'appareil de mesure de couple du genou pendant les contractions statiques chez les êtres humains alités selon l'une quelconque des revendications qui précèdent, **caractérisé par le fait que**
- la plaque d'embase (2) est constituée d'un matériau dur non-flexible, tel que de l'aluminium ou un autre matériau métallique, où la longueur et la largeur privilégiées de la plaque d'embase (2) se situent entre 50 et 70 cm, idéalement 60 cm, et entre 25 et 35 cm, idéalement 30 cm, respectivement ; et en ce que
- l'élément horizontal long (1) fait environ 1 m de long et est constitué d'un profilé en aluminium unique, tandis que les éléments plus courts (4, 5) sont des profilés en aluminium mesurant 0,5 m de longueur.

9. L'appareil de mesure de couple du genou pendant les contractions statiques chez les êtres humains alités selon l'une quelconque des revendications précédentes, **caractérisé par le fait que** le support de genou (16) peut être fixe ou peut être de préférence mobile en utilisant des fixations rotatives connues, de façon à ce qu'un angle de genou adéquat puisse être réglé ; et en ce que le support de genou (16) est fourni avec la sangle (18) fixée près de la jonction des deux éléments plus courts (4, 5) pour fixer le genou fermement contre l'élément de support cylindrique (16).

10. L'appareil de mesure de couple du genou pendant les contractions statiques chez les êtres humains alités selon l'une quelconque des revendications qui précèdent, **caractérisé par le fait que** l'attelle du tibia a deux surfaces légèrement incurvées, dont les côtés concaves se font face, où la distance entre les surfaces est ajustable, ce qui permet au tibia d'être saisi au niveau de la partie distale juste au-dessus de la cheville.

11. L'appareil de mesure de couple du genou pendant les contractions statiques chez les êtres humains alités selon l'une quelconque des revendications qui précèdent, **caractérisé par le fait que** le capteur de couple peut être tout capteur approprié pour mesurer le couple, et est réglé de façon à envoyer des données au boîtier électronique principal pendant la prise de mesures, où ledit boîtier électronique principal (23) comprend :
- un commutateur marche/arrêt (25),
- des ports d'entrée/sortie (27) tels qu'un port USB,
- une antenne sans fil (26), qui permet de transmettre les données sans fil, et
- un connecteur de sortie de signal brut (28),
et est réglé pour amplifier les signaux du capteur de couple, en effectuant une conversion analogique-numérique, faisant l'acquisition des données à 1000 Hz et transmettant les signaux via un module Bluetooth à un ordinateur.

12. L'appareil de mesure de couple du genou pendant les contractions statiques chez les êtres humains alités selon l'une quelconque des revendications qui précèdent, **caractérisé par le fait que** l'appareil peut également comprendre des plaques supplémentaires raccordées à l'élément long horizontal du cadre à dynamomètre.

13. Une méthode d'utilisation de l'appareil selon l'une quelconque des revendications qui précèdent, **caractérisée par le fait qu'**un patient est positionné et maintenu en place de la façon suivante :
- les hanches/la zone du bassin du patient reposent sur la plaque d'embase (2), et le patient est couché sur le dos,
- le cadre de fixation (9, 10, 11) maintient fermement en place les hanches du patient contre la plaque d'embase,
- l'élément horizontal plus long (1) se poursuit horizontalement depuis l'extrémité de la plaque d'embase (2) sur le côté de la jambe testée,
- les deux éléments plus courts (4, 5) sont disposés de façon à former un triangle, où le premier élément plus court (4) commence au niveau de la partie distale de l'élément de cadre plus long, et pointe en direction de la partie supérieure du corps du patient, et la jonction des deux éléments plus courts se fait au niveau de l'articulation du genou, tandis que le second élément plus court (5) pointe à l'opposé du sujet,
- la partie distale du tibia est maintenue par l'attelle de fixation,
- le genou est maintenu en place contre l'élément cylindrique (16) avec la sangle de fixation (18),
- les hanches et les articulations des genoux du patient sont en position fléchie (environ 45° chacune, avec des ajustements possibles).

14. Une méthode d'utilisation de l'appareil selon la revendication précédente, **caractérisée par le fait que** les prises de mesure sont effectuées de la façon suivante :
- le patient fléchit et déploie le genou ou l'activité musculaire est déclenchée de façon externe en utilisant les méthodes connues de stimulation d'un nerf de l'aine ou d'un muscle de la cuisse, où le capteur mesure le couple créé,
- la flexion et l'extension peuvent être répétées plusieurs fois afin d'obtenir une prise de mesure plus fiable,
- les données provenant du capteur sont envoyées au boîtier électronique principal et éventuellement sur un ordinateur pour être traitées et analysées.

15. L'appareil selon l'une quelconque des revendications 1 à 12, **caractérisé par le fait qu'**il est intégré dans le châlit d'un lit.
